(19) Europäisches Patentamt
European Patent Office
Office européen des brevets

(11) Numéro de publication: **0 138 716**
**B1**

(12) # FASCICULE DE BREVET EUROPÉEN

(45) Date de publication du fascicule du brevet: **12.08.87**

(51) Int. Cl.⁴: **C 07 D 265/30**, A 61 K 31/535

(21) Numéro de dépôt: **84402067.7**

(22) Date de dépôt: **12.10.84**

(54) **Nouveaux dérivés de 2-tolyl-morpholine utiles en thérapeutique.**

(30) Priorité: **14.10.83 FR 8316406**

(43) Date de publication de la demande: **24.04.85 Bulletin 85/17**

(45) Mention de la délivrance du brevet: **12.08.87 Bulletin 87/33**

(84) Etats contractants désignés: **AT BE CH DE FR GB IT LI LU NL SE**

(56) Documents cité:
**EP-A-0 080 940**
**GB-A-851 311**
**US-A-2 835 669**

**EUROPEAN JOURNAL OF MEDICINAL CHEMISTRY - CHIMICA THERAPEUTICA, vol. 11, no. 3, mai/juin 1976, pages 201-207; N. BUSCH et al.: "Tétrahydrooxazines-1,4. I. Nouvelle méthode de synthèse et étude de leur interaction avec les récepteurs tryptaminergiques D"**

(73) Titulaire: **LABORATOIRE L. LAFON Société anonyme dite:, 1 rue Georges Médéric, F-94701 Maisons- Alfort (FR)**

(72) Inventeur: **Lafon, Louis, 5, rue de l'Alboni, F-75016 Paris (FR)**

(74) Mandataire: **Clisci, Serge, S.A. FEDIT- LORIOT CONSEILS EN PROPRIETE INDUSTRIELLE 38, avenue Hoche, F-75008 Paris (FR)**

**EP 0 138 716 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

Présente invention a trait à de nouveaux produits industriels appartenant à la famille des dérivés de 2-phényl-morpholine, à savoir les dérivés de 2-tolyl-morpholine décrits ci-après. Elle a également trait à l'utilisation de ces nouveaux produits en thérapeutique, notamment en tant qu'agents actifs sur le système nerveux central (SNC).

On sait que l'on a déja décrit un certain nombre de composés appartenant à la famille des dérivés de 2-phényl-morpholine et étudié leurs propriétés pharmacologiques; voir à cet effet GB-A-851 311, US-A-2 835 669, US-A-2 997 469, FR-A-7443 M, FR-A-1 535 615, FR-A-2 111 882, FR-A-2 471 378, EP-A-80940 et l'article de N. BUSCH et al. Eur. J. Med. Chem. Chimica Thérapeutica 11 (N°3), pages 201-207 (1976), où lesdits dérivés de 2-phényl-morpholine sont proposés ou envisagés en tant qu'agents excitants, stimulants, tranquillisants, sédatifs, anti-inflammatoires, analgésiques et/ou hypotenseurs.

On vient de trouver de façon surprenante que les composés selon l'invention, qui ont tous des effets sédatifs, sont particuliérement intéressants en thérapeutique en tant que substances sédatives et stimulantes du SNC, d'une part, et/ou vasodilatatrices, d'autre part, eu égard à l'enseignement de l'art antérieur précité. En particulier, les composés selon l'invention sont plus intéressants que la 2-o-tolyl-4-méthyl-morpholine décrite dans EP-A-80940 précité. De plus les composés selon l'invention ne sont pas tératogènes, à la différence de certains dérivés de 2-phényl-morpholine antérieurement connus.

Les nouveaux dérivés de 2-tolyl-morpholine selon l'invention qui répondent à la formule générale

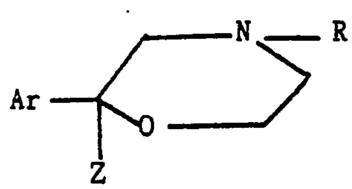

(I)

(où Ar est un groupe tolyle ou trifluorométhylphényle, Z est H ou OH, et R est un groupe alkyle en $C_1$-$C_4$) sont caractérisés en ce qu'ils sont choisis parmi l'ensemble des 2-tolyl-4-alkyl-morpholines constitué par:

a) la 2-p-tolyl-4-isopropyl-morpholine et ses sels d'addition,
b) la 2-m-tolyl-4-méthyl-morpholine et ses sels d'addition,
c) la-p-tolyl-4-méthyl-morpholine et ses sels d'addition,
d) la 2-o-tolyl-4-tertiobutyl-morpholine et ses sels d'addition,
e) la 2-p-tolyl-4-tertiobutyl-morpholine et ses sels d'ad dition, et,
f) la 2-(m-trifluorométhylphényl)-2-hydroxy-4-éthyl-morpholine et ses sels d'addition.

Par sels d'additions, on entend ici, d'une part les sels d'addition d'acide obtenus par réaction des bases libres selon l'invention avec les acides minéraux et organiques, et, d'autre part, les sels d'ammonium. Parmi les acides utilisables pour salifier les bases libres selon l'invention, on peut notamment mentionner les acides chlorhydrique, bromhydrique, acétique, formique, propionique, oxalique, fumarique, maléique, succinique, benzoique, cinnamique, mandélique, citrique, malique, tartrique, aspartique, glutamique, méthanesulfonique, p-toluènesulfonique. Parmi les composés permettant d'obtenir des sels d'ammonium, on peut notamment citer $ICH_3$ et $ClCH_3$.

Les sels d'addition d'acides sont préférés aux sels d'ammonium. Parmi les sels d'addition d'acides les chlorhydrates, donnés de façon non limitative dans le tableau I ci-après, sont les sels les plus intéressants à l'heure actuelle sur le plan neuropsychopharmacologique.

Les composés selon l'invention peuvent être préparés selon une méthode connue en soi par application de mécanismes réactionnels classiques. Le procédé, que l'on préconise ici pour préparer les composés Z; H, consiste à cycliser un 2-(N-alkyl-N-β -hydroxyéthyl)-amino-1-tolyl-1-éthanol de formule

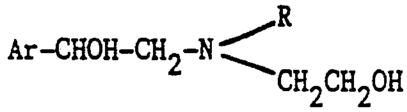

(II)

où Ar et R sont définis comme ci-dessus) au moyen de $H_2SO_4$ concentré, à une température comprise entre 0 et 25°C, pendant au moins 1 heure.

De façon avantageuse cette cyclisation est mise en oeuvre avec de l'acide sulfurique concentré ayant une densité de 1,84.

La synthése totale des composés selon l'invention, où Z est H à partir d'un bromure de méthylphénacyle de formule (III) et d'une N-β-hydroxyéthyl-alkylamine (IV), est schématisée dans le diagramme A ci-après.

**DIAGRAMME A**

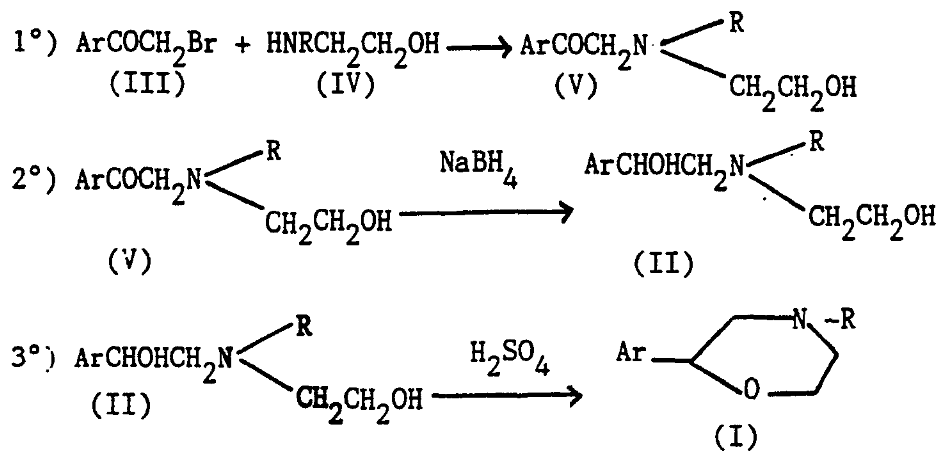


Le procédé, que l'on préconise ici pour préparer le composé Z = OH selon l'invention, consiste à faire réagir l'α-bromo-m-trifluorométhyl-acétophénone (autre nomenclature: bromure de m-trifluorométhyl-phénacyle) de formule

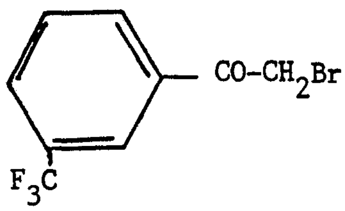

(III Bis)

avec un excès par rapport aux conditions stoechiométriques de N-β-hydroxyéthyl-alkylamine (IV) (où R est éthyle), dans un solvant inerte, notamment l'éther diméthylique ou diéthylique. De façon avantageuse on utilisera environ 2 moles de (III bis) pour 1 mole de (IV), la durée de réaction étant comprise entre 4 et 24 h, et la température entre 5 et 25° C.

Selon l'invention, on préconise une composition thérapeutique qui est caractérisée en ce qu'elle renferme, en association avec un excipient physiologiquement acceptable au moins un dérivé de 2-tolyl-4-alkyl-morpholine choisi parmi l'ensemble constitué par les 2-p-tolyl-4-isopropyl-morpholine, 2-m-tolyl-4-méthyl-morpholine, 2-p-tolyl-4-méthyl-morpholine, 2-o-tolyl-4-tertiobutyl-morpholine, 2-p-tolyl-4-tertiobutyl-morpholine, 2-(m-trifluorométhyl-phényl)-2-hydroxy-4-éthyl-morpholine, et leurs sels d'addition, en tant que principe actif.

Bien entendu, dans une telle composition, le principe actif, qui est choisi parmi l'ensemble des composés selon l'invention et leurs sels non toxiques, intervient en quantité pharmaceutiquement efficace.

Les composés selon l'invention possèdent la propriété commune d'agir en tant que sédatifs.

Les CRL 40875 (exemple I), CRL 40912 (exemple 2) et CRL 40897 (exemple 3) présentent d'une manière générale des effets sédatifs (hypomotilité, hypothermie, diminution de l'agressivité intergroupes) et des effets stimulants notamment du type α-adrénergique. En bref, à faible dose, ils se comportent comme des sédatifs et à forte dose comme des antidépresseurs. Ils se distinguent de leur analogue, décrit dans EP-A-80940, le CRL 40915 (CP-1), par la variation de leurs effets sédatifs. Plus précisément, on a constaté que pour les CRL 40875, CRL 40912 et CRL 40897 les effets sédatifs augmentaient de façon continue avec la dose administrée jusqu'à une valeur maximale, puis décroissaient de façon continue au fur et à mesure que la dose augmentait quand les effets stimulants se manifèstaient, et que, en revanche, pour le CRL 40915, les effets sédatifs n'apparaissaient pas de façon continue ou progressive quand la dose administrée augmentait (voir à cet effet EP-A-80940 précité, page 13 lignes 6-10) mais se produisaient en tout ou rien: "pas de sédation à une dose donnée, sédation importante à la dose immédiatement supérieure utilisé".

Les CRL 40903 (exemple 4), CRL 40910 (exemple 5) et CRL 41092 (exemple 6) ont des effets sédatifs et sont surtout intéressants en tant que vasodilatateurs eu égard à leurs propriétés cardiovasculaires.

D'autres avantages et caractéristiques de l'invention seront mieux compris à la lecture qui va suivre d'exemples de préparation et de résultats d'essais pharmacologiques, l'ensemble de ces éléments n'étant nullement limitatif mais donné à titre d'illustration.

**PREPARATION I**

Obtention du chlorhydrate de 2-p-tolyl-4-isopropyl-morpholine (exemple 1; N° de code: CRL 40875)

On coule, goutte é goutte 100 g (0,469 mole) de bromure de p-méthylphénacyle, dans une solution constituée par 193,23 g (1,876 mole) de 2-isopropylamino-1-éthanol et 500 ml de méthanol. On porte au reflux pendant 3 heures, évapore à sec, reprend le résidu d'évaporation avec 500 ml d'eau, extrait à l'acétate d'éthyle, lave la phase acétate d'éthyle avec de l'eau, sèche sur $MgSO_4$, filtre pour éliminer $MgSO_4$ et évapore à sec.

Le résidu d'évaporation ainsi obtenu, qui renferme la N-β-hydroxyéthyl-N-(4-méthylphénacyl)-isopropylamine, est repris avec 1500 ml de méthanol. On refroidit jusqu'à - 5°C et ajoute 38 g (1 mole) de $NaBH_4$. On laisse en contact pendant 8-12 h à -5°C, puis on ajoute 60 ml d'acide acétique, évapore à sec, reprend le résidu d'évaporation avec de l'eau, ajuste le pH à 11 avec NaOH, extrait à l'acétate d'éthyle, lave la phase acétate d'éthyle avec de l'eau, puis extrait ladite phase acétate d'éthyle avec un mélange de 250 ml d'eau et 50 ml d'acide HCl concentré (d=1,19). La phase aqueuse résultante est lavée à l'éther diéthylique additionnée de NaOH jusqu'à pH 11, extraite à l'acétate d'éthyle, la phase acétate d'éthyle ainsi obtenue étant lavée é l'eau, séchée sur $MgSO_4$, filtrée puis évaporée à sec.

Le résidu d'évaporation ainsi obtenu, qui renferme le 2-(N-β-hydroxyéthyl-N-isopropyl)-amino-1-p-tolyl-1-éthanol, est coulé dans 76 ml d'acide sulfurique (d=1,84) en refroidissant le milieu réactionnel au moyen d'un bain de glace. L'addition terminée, on laisse en contact à 5-20°C pendant 1 heure, puis verse le milieu réactionnel dans un mélange de 500 ml d'eau, de glace et de 274 ml de NaOH 10N. On extrait à l'acétate d'éthyle, lave à l'eau, sèche au $MgSO_4$ et filtre. A partir du filtrat on précipite le chlorhydrate attendu au moyen d'éthanol chlorhydrique. Par recristallisation dans le mélange acétone-éthanol (1: 1) v/v on obtient 38,7 g (rendement global: 32 %) de CRL 40875. F = 225°C (avec décomposition) analyse:

% Cl mesuré = 14,08 %
% Cl théorique = 13,89 %

**PREPARATION II**

Obtention du chlorhydrate de 2-m-tolyl-4-méthyl-morpholine (exemple 2, N° de Code: CRL 40912)

On dissout 67 g (0,5 mole) de métaméthylacétophénone dans 100 ml d'acide acétique. On refroidit au bain de glace et on coule 25,8 ml de brome. On laisse en contact une heure et évapore à sec.

On reprend le résidu d'évaporation. ainsi obtenu, qui renferme le bromure de m-méthylphénacyle, par l'acétone et coule cette solution dans une solution de 2,5 moles (187,5 g) de 2-méthylamino-1-éthanol dans 500 ml de méthanol. On porte à reflux 4 heures, évapore à sec, reprend à l'eau et extrait à l'acétate d'éthyle. On lave la phase organique à l'eau, l'extrait par un mélange de 500 ml d'eau et 60 ml d'HCl concentré (d=1,19); lave la phase aqueuse à l'acétate d'éthyle, la neutralise jusqu'à pH = 11 par $K_2CO_3$, extrait à l'acétate d'éthyle lave à l'eau et séche le solvant. On évapore a sec.

On reprend le résidu d'évaporation ainsi obtenu, qui renferme la N-β-hydroxyéthyl-N-(3-méthylphénacyl)-méthylamine par 500 ml de méthanol. On ajoute 38 g (1 mole) de borohydrure de sodium. On laisse en contact une nuit. On détruit par 60 ml d'acide acétique, évapore à sec, reprend à l'eau, extrait è l'acétate d'éthyle, lave le solvant et le séche par $MgSO_4$. On filtre et évapore à sec.

On coule le résidu d'évaporation ainsi obtenu, qui renferme, le 2-(N-β-hydroxyéthyl-N-méthyl)-amino-1-(m-tolyl)-1-éthanol, dans 125 ml d'acide sulfurique concentré (d=1,84), en refroidissant au moyen d'un bain de glace. On laisse en contact une heure, jette le mélange réactionnel dans un mélange de 500 ml d'eau, de glace et de 460 ml de NaOH 10 N. On extrait à l'éther, lave l'éther à l'eau, sèche l'éther sur $MgSO_4$ puis précipite le chlorhydrate attendu au moyen d'éthanol chlorhydrique. Par recristallisation dans le mélange acétone-éthanol (1: 1)v/v, on obtient 20 g (rendement global: 18 %) de CRL 40912.

F = l59°C.
analyse:
% Cl mesuré = 15,77 %
% Cl théorique = 15,60 %

**PREPARATION III**

Obtention du chlorhydrate de 2-p-tolyl-4-méthyl-morpholine (exemple 3: N° de Code: CRL 40897)

En procédant comme indiqué dans la préparation I mais en remplaçant la N-β-hydroxyéthyl-isopropylamine par la N-β-hydroxyéthylméthylamine, on obtient le CRL 40 897 (avec un rendement global de 23 %). F = 186 °C.

Analyse:
% $Cl^-$ mesuré = 16,16 %
% $Cl^-$ théorique = 15,60 %

**PREPARATION IV**

Obtention du chlorhydrate de 2-p-tolyl-4-tertiobutyl-morpholine (exemple 4; N° de Code: CRL 40 903)

On coule goutte à goutte une solution de 36,64 g (0,172 mole) de bromure de p-méthylphénacyle dans de l'acétone, dans une solution de 80,4 g (0,887 mole) de 2-tertiobutylamino-1-éthanol dans le méthanol.

On porte à reflux pendant 4 heures, évapore à sec, reprend à l'eau le résidu d'évaporation, extrait avec de l'acétate d'éthyle, lave le solvant à l'eau, extrait le solvant par 200 ml d'eau contenant 20 ml d'acide chlorhydrique concentré, lave la phase aqueuse avec de l'acétate d'éthyle, neutralise par de la soude, extrait à nouveau à l'acétate d'éthyle, lave à l'eau, sèche le solvant sur du sulfate de magnésium et évapore à sec.

Le produit sec obtenu est repris dans 500 ml de méthanol anhydre et on y ajoute 10 g de borohydrure de sodium. On laisse en contact plusieurs heures pour réaliser la réaction de réduction; on ajoute alors 20 ml d'acide acétique puis on évapore à sec, extrait à l'acétate d'éthyle, lave le solvant à l'eau et le sèche sur du sulfate de magnésium. Puis on évapore à sec. Le résidu d'évaporation ainsi obtenu est alors coulé dans 50 ml d'acide sulfurique concentré de façon à réaliser la cyclisation. On laisse le mélange résultant reposer pendant une heure et on le verse dans un mélange contenant 500 ml d'eau, 250 ml de soude 10 N et de la glace.

Le mélange ainsi obtenu est extrait à l'éther, on lave la phase éthérée à l'eau, et sèche sur du sulfate de magnésium. On précipite le chlorhydrate attendu par adjonction d'éthanol chlorhydrique et purifie par recristallisation dans le mélange étheréthanol (1: 1) v/v. On obtient ainsi 10 g de CRL 40903.

F = 228°C.

Analyse:

% $Cl^-$ mesuré = 13,32 %

% $Cl^-$ théorique = 13,17 %

**PREPARATION V**

Obtention du chlorhydrate de 2-(m-trifluorométhylphényl)-2-hydroxy-4-éthyl-morpholine (exemple 6; N° de Code: CRL 41092)

On dissout 38 g (0,19 mole) de métatrifluorométhylacétophénone dans 300 ml d'éther et on y coule goutte à goutte 30,64 g (0,19 mole) de brome. On laisse réagir pendant environ 1 heure, on évapore à sec et laisse dans un dessiccateur sous vide, en présence de KOH, pour compléter le séchage. Le résidu ainsi obtenu est repris dans 300 ml d'éther et on coule la solution résultante dans une solution de 34,08 g (0,38 mole) de 2-éthylamino-éthanol dans 400 ml d'éther. On laisse réagir sous agitation pendant 24 heures. On réalise ensuite les opérations suivantes: filtration de l'insoluble, lavage à l'éther, lavage à l'eau, extraction de la phase éthérée par 200 ml d'eau contenant 25 ml d'acide chlorhydrique concentré, lave la phase aqueuse à l'éther, neutralise jusqu'à pH 11 par de la soude, extrait à l'éther qu'on lave à l'eau et sèche. On précipite le chlorhydrate attendu par adjonction d'éthanol chlorhydrique. Par recristallisation dans l'acétone-éthanol (1:1) v/v, on obtient 23 g de CRL 41 092. F = 194°C.

Analyse:

% $Cl^-$ mesuré: 11,90 %

% $Cl^-$ théorique: 11,40 %

On a résumé ci-après les résultats des essais qui ont été entrepris.

**ESSAIS RELATIFS AU CRL 40875 (EXEMPLE 1)**

A. **ETUDE CARDIOVASCULAIRE**

Les résultats des essais cardiovasculaires ont mis en évidence que le CRL 40875 est hypotenseur et tachycardisant chez le chien. Chez le rat normalement hypertendu, le CRL 40875 ne modifie pas les paramètres cardiovasculaires.

B. **ETUDE NEUROPSYCHOPHARMACOLOGIQUE**

Dans les essais qui suivent le CRL 40875, en solution dans de l'eau distillée, a été administré par voie intrapéritonéale sous un volume de 20 ml/kg chez la souris mâle et de 5 ml/kg chez le rat mâle.

I - **PRETOXICITE**

On constate que sur des lots de trois souris, le CRL 40875 provoque à la dose de 128 mg/kg, une salivation épaisse, des convulsions puis la mort (des 3 animaux du lot) en 10 minutes, et aux doses de 64 mg/kg et 32 mg/kg des crampes abdominales, des tremblements, mais aucune mortalité. La DL-O (dose maximale non mortelle) est donc supérieure à 64 mg/kg I.P. et inférieure à 128 mg/kg I.P. chez la souris mâle.

II - **COMPORTEMENT GLOBAL ET REACTIVITES**

Des lots de trois souris sont observés avant puis 15 minutes, 30 minutes, 1 heure, 2 heures, 3 heures et 24 heures après administration de CRL 40 875. On constate que:

- la dose de 32 mg/kg provoque:

- une hypothermie (-4,1°C) pendant 2 à 3 heures,

- une diminution de la réactivité au toucher et du tonus musculaire pendant 2 heures,
- une piloérection (2 animaux sur 3) pendant 2 à 3 heures,
- une mydriase modérée pendant 0,5 heure;
- la dose de 8 mg/k provoque:
- une hypothermie (-3,9° C) pendant 1 heure,
- une piloérection (2/3) pendant 3 heures, et,
- la dose de 2 mg/kg provoque:
- une hypothermie modérée (-1,5 ° C) pendant 0,5 heure.

III. **INTERACTION AVEC L'APOMORPHINE**

1. - chez la souris

Des lots de 6 souris reçoivent le CRL 40 875, 0,5 heure avant l'injection sous cutanée de 1 ou 16 mg/kg d'apomorphine.

On constate que le CRL 40 875 exerce un effet hypothermisant intrinséque et s'oppose partiellement à forte dose (32 mg/kg) à l'hypothermie induite par 16 mg/kg d'apomorphine, et qu'il ne modifie pas le comportement de verticalisation et les stéréotypies.

2. - chez le rat

Le CRL 40 875 est administré à des lots de 6 rats 0,5 heure avant l'injection sous cutanée de 0,5 mg/kg d'apomorphine.

On constate que le CRL n'altère pas de façon notable le comportement stéréotypé induit par l'apomorphine chez le rat.

IV. **INTERACTION AVEC L'AMPHETAMINE**

L'amphétamine (2 mg/kg) est injectée par voie intrapéritonéale à des lots de 6 rats, une demi-heure après l'administration de CRL 40 875, on observe que les stéréotypies amphétaminiques ne sont pas modifiées par le CRL 40 875.

V. **INTERACTION AVEC LA RESERPINE**

Quatre heures après l'injection intrapéritonéale de 2,5 mg/kg de réserpine, des lots de 6 souris reçoivent le CRL 40 875. On observe les effets sur la température et le ptôsis. On constate que le CRL 40 875 aggrave à forte dose l'hypothermie réserpinique sans modifier le ptôsis réserpinique.

VI. **INTERACTION AVEC L'OXOTREMORINE**

Le CRL 40 875 est administré à des lots de 6 souris une demi-heure avant l'injection intrapéritonéale de 0,5 mg/kg d'oxotrémorine.

1. - Action sur la température

Le CRL 40 875 exerce un effet hypothermisant et, à forte dose, s'oppose partiellement à la baisse de température provoquée par l'oxotremorine.

2. - Action sur les tremblements

Aux doses de 8 et 32 mg/kg, le CRL 40 875 semble augmenter la durée des tremblements dûs à l'oxotrémorine.

3. - Action sur les symptômes cholinergiques périphériques

Aux doses de 0,5 mg/kg, 2 mg/kg, 8 mg/kg et 32 mg/kg, le CRL 40 875 accroît la proportion de souris qui présentent de la défécation à la suite de l'administration d'oxotrémorine.

VII. **ACTION SUR LE TEST DES SUATRE PLAQUES, LA TRACTION ET L'ELECTRO-CHOC.**

Le test est pratiqué sur des lots de 10 souris une demi-heure après l'administration de CRL 40 875.

On observe que le CRL 40 875 n'entraîne pas d'augmentation du nombre de passages punis, qu'il ne provoque pas d'incapacité motrice majeure et qu'il ne modifie pas les effets convulsivants de l'électrochoc.

VIII. **ACTION SUR LA MOTILITE SPONTANEE**

Une demi-heure après avoir reçu le CRL 40 875 les souris (6 par dose, 12 témoins) sont placées en actimétre où leur motilité est enregistrée pendant 30 minutes.

On constate que dès la dose de 2 mg/kg, le CRL 40 875 entraîne une hypomotilité qui devient trés importante aux doses de 8 et 32 mg/kg.

IX. **ACTION SUR L'AGRESSIVITE INTERGROUPES**

Après avoir séjourné pendant 3 semaines dans chacune des moitiés d'une cage séparées par une cloison opaque, des groupes de 3 souris reçoivent le CRL 40 875. Une demi-heure plus tard, les deux groupes d'une même cage sont réunis par retrait de la cloison et on note le nombre de combats qui surviennent en 10 minutes.

On observe que, aux doses utilisées (0,5 mg/kg et 2 mg/kg) qui n'exercent qu'un effet modéré sur l'activité motrice, le CRL 40 875 entraîne une suppression des combats.

X. **ACTION VIS-A-VIS DE QUELQUES COMPORTEMENTS PERTURBES PAR DIVERS AGENTS**

1. Motilité réduite par habituation à l'enceinte

Après 18 heures de séjour dans les actimétres, les souris (6 par dose, 12 témoins) reçoivent le CRL 40 875. Elles sont aussitôt replacées dans leurs enceintes respectives et, une demiheure plus tard, on enregistre leur motilité pendant 30 minutes.

On observe que le CRL 40 875 n'entraîne pas de reprise de l'activité chez la souris habituée à son enceinte.

2. Motilité réduite par agression hypoxique

Une demi-heure après avoir reçu le CRL 40 875 les souris (10 par dose, 20 témoins) sont soumises à une anoxie hypobare aiguë [dépression de 600 mm Hg (soit environ 8 x $10^4$ pascals) en 90 secondes; détente de 45

secondes], puis elles sont placées en actimètre où leur motilité est enregistrée pendant 10 minutes.

On constate que le CRL 40 875 ne provoque pas d'amélioration de la récupération motrice chez la souris dont la motilité a été déprimée à la suite d'un séjour bref dans une enceinte a pression réduite.

3. -Anoxie asphyxique

Des lots de 20 souris reçoivent le CRL 40 875 une demi-heure avant l'administration intrapéritonéale de 32 mg/kg de triiodoéthylate de gallamine (qui est un agent curarisant de référence).

On observe que, aux doses utilisées, le CRL 40 875 retarde l'apparition des convulsions et de la mort consécutives à une anoxie asphyxique provoquée par un curarisant.

XI. **CONCLUSION**

L'étude neuropsychopharmacologique du CRL 40 875 montre un certain nombre d'effets sédatifs (hyporéactivité, hypothermie, hypomotilité, diminution importante de l'agressivité, retard d'apparition des convulsions asphyxiques) aux faibles doses et des effets antidépresseurs (antagonismes des hypothermies induites par l'apomorphine et l'oxotrémorine) aux fortes doses. On note par ailleurs que le CRL 40 875 présente des signes de stimulation α-adrénergique périphérique (salivation, piloérection, tremblements).

**ESSAIS RELATIFS AU CRL 40 897 (EXEMPLE 3)**

- **ETUDE NEUROPSYCHOPHARMACOLOGIQUE**

Dans les essais qui suivent, le CRL 40 897 en solution dans de l'eau distillée (pH 4) a été administré par voie intrapéritonéale sous un volume de 20 ml/kg chez la souris mâle, et, sous un volume de 5 ml/kg chez le rat mâle, les modalités opératoires étant celles données ci-dessus pour le CRL 40 875.

I. **PRETOXICITE**

Sur des lots de trois souris mâles chacun, on observe que le CRL 40 897 provoque, par voie I.P., à la dose de 256 mg/kg, une dyspnée, une salivation abondante (pour 1 animal sur 3), des convulsions (2/3) et la mort (3/3) qui intervient en 10 minutes; à la dose de 128 mg/kg, une dyspnée, des tremblements, des convulsions (2/3), une sédation mais aucune mortalité; et, à la dose de 64 mg/kg, une dyspnée une sédation et des tremblements (1/3). La DL-O est donc supérieure à 128 mg/kg I.P. et inférieure à 256 mg/kg I.P. chez la souris mâle.

II. **COMPORTEMENT GLOBAL ET REACTIVITES**

On constate que le CRL 40 897 étudié sur des lots de trois souris chacun, provoque:

- à la dose de 64 mg/kg
- une sédation (3 animaux sur 3) pendant 3 heures,
- une respiration déprimée (2/3) pendant 3 heures,
- une salivation importante (3/3) pendant 1 heure,
- une hypothermie (-2,2° C) pendant 1 à 2 heures,
- une mydriase pendant 1 à 2 heures;
- à la dose de 16 mg/kg
- une hypothermie (-2,4° C) pendant 0,5 h; et,
- à la dose de 4 mg/kg
- une hypothermie (-2,2° C) pendant 0,5 heure

III. **INTERACTION AVEC L'APOMORPHINE**

1. chez la souris

On observe que malgré un effet hypothermisant présent dès 4 mg/kg, le CRL 40 897 à dose élevée (64 mg/kg) combat partiellement la baisse de température induite par l'apomorphine sans modifier le comportement de verticalisation et les stéréotypies.

2. chez le rat

On constate que le CRL 40 897 ne modifie pas les stéréotypies induites par l'apomorphine.

IV. **INTERACTION AVEC L'AMPHETAMINE**

Le lot témoin de 6 rats présente un index de stéréotypies bas en raison de l'absence totale de reprise à l'amphétamine d'un des six rats. Les augmentations observées dans les autres lots ne sont que le reflet de cette anomalie.

V. **INTERACTION AVEC LA RESERPINE**

On observe que le CRL 40 897 ne modifie pas l'hypothermie et le ptôsis induits par la réserpine.

VI. **INTERACTION AVEC L'OXOTREMORINE**

1. - Action sur la température

On observe que malgré un effet hypothermisant net dès 4 mg/kg, le CRL 40 897 à dose élevée (64 mg/kg) combat partiellement la baisse de température induite par l'oxotrémorine.

2. - Action sur les tremblements

On constate que les tremblements dûs à l'oxotrémorine ne sont pas influencés par le CRL 40 897.

3. Action, sur les symptômes cholinergiques périphériques

On observe que le CRL 40 897 laisse inchangés les signes de stimulation cholinergique périphérique induits par l'oxotrémorine.

VII. **ACTION SUR LE TEST DES QUATRE PLAQUES, LA TRACTION ET L'ELECTROCHOC**

On observe que le CRL 40 897 n'entraîne pas d'augmentation du nombre de passages punis, qu'il ne

provoque pas d'incapacité motrice majeure et qu'il ne modifie pas les effets convulsivants et létaux de l'électrochoc.

VIII. **ACTION SUR LA MOTILITE SPONTANEE**

On note que dès la dose de 4 mg/kg, le CRL 40 897 déprime l'activité motrice spontanée de la souris

IX. **ACTION SUR L'AGRESSIVITE INTERGROUPES**

On constate que le CRL 40 897 n'entraîne pas de diminution nette du nombre de combats.

X. **ACTION VIS-A-VIS DE QUELQUES COMPORTEMENTS PERTURBES PAR DIVERS AGENTS**

1. Motilité réduite par habituation à l'enceinte

Le CRL 40 897 ne provoque pas de reprise de l'activité chez la souris habituée à son enceinte.

2. - Motilité réduite par agression hypoxique

On observe que le CRL 40 897 n'entraîne aucune amélioration de la récupération motrice chez les souris dont la motilité a été déprimée é la suite d'un séjour bref dans une enceinte à pression réduite.

3. - Anoxie asphyxique

Aux doses étudiées, on constate que, le CRL 40 897 retarde l'apparition des convulsions et de la mort consécutives à une anoxie asphyxique provoquée par le curarisant de référence (le triiodoéthylate de gallamine).

**ESSAIS RELATIFS AU CRL 40 912 (EXEMPLE 2)**

A. **ETUDE CARDIOVASCULAIRE**

Chez le chien anesthésié, on constate que le CRL 40 912 administré par voie intrapéritonéale est hypertenseur à la dose de 1 mg/kg, que cet effet diminue à la dose de 5 mg/kg et que l'on a une bradycardie à partir de 5 mg/kg.

Chez le rat éveillé, on note que le CRL 40 912 n'a aucune action sur la pression artérielle moyenne et que la bradycardie est de courte durée.

On constate par ailleurs que le -CRL 40 912 présente un effet du type α-stimulant.

B. **ETUDE NEUROPSYCHOPHARMACOLOGIQUE**

Dans les essais qui ont été entrepris, le CRL 40 912 en solution dans l'eau distillée (pH 4,5) a été administré par voie intrapéritonéale, sous un volume de 20 ml/kg chez la souris mâle et sous un volume de 5 ml/kg chez le rat mâle, les modalités opératoires étant celles données ci-dessus pour le CRL 40 875.

On constate que

- la DL-O chez la souris mâle est supérieure à 128 mg/kg et inférieure à 256 mg/kg par voie I.P.;
- le CRL 40 912 présente une action stimulante α-adrénergique périphérique très nette (piloérection, exophtalmie, antagonisme important du ptôsis réserpinique)
- en revanche, au niveau central, le CRL 40 912 présente des effets sédatifs (hypomotilité, hypothermie) comparables à ceux des substances bloquantes α-adrénergiques ou à la clonidine (qui est un stimulant des récepteurs α-présynaptiques); ces effets sédatifs rendent compte de la diminution de l'agressivité et du retard d'apparition des convulsions et de la mort après anoxie asphyxique;
- toutefois, le CRL 40 912 ne diminue pas l'intensité des stéréotypies amphétaminiques (à la différence des α-bloquants et de la clonidine) et ne provoque pas de reprise de l'activé motrice chez la souris habituée à son enceinte (à la différence de la clonidine)
- enfin, à forte dose, le CRL 40 912 s'oppose aux effets hypothermisants de l'apomorphine et de l'oxotrémorine, et, à dose plus faible à ceux de la réserpine (à la différence des α-bloquants et de la clonidine).

En bref, le CRL 40 912 agit en tant que sédatif à faible dose, puis en tant qu'antidépresseur à forte dose, mais son mécanisme d'action dans l'organisme est parfois différent de celui des α-bloquants et de la clonidine.

**ESSAIS RELATIFS AUX CRL 40 903 (EXEMPLE 4), CRL 40 910 (EXEMPLE 5) ET CRL 41 092 (EXEMPLE 6)**

Les études toxicologiques et neuropsychopharmacologiques entreprises selon les modalités opératoires décrites ci-dessus pour le CRL 40 875 ont permis les observations suivantes:

- CRL 40 903:
- la DL-O est supérieure à 128 mg/kg par voie I.P. chez la souris mâle,
- les doses de 64 mg/kg et 128 mg/kg par voie I.P. chez la souris mâle et de 32 mg/kg par voie I.P. chez le rat mâle induisent une sédation, une respiration déprimée;
- CRL 40 910:
- la DL-O est supérieure à 128 mg/kg par voie I.P. chez la souris mâle,
- la dose de 64 mg/kg par voie I.P. chez la souris mâle donne une sédation (0,5 à 3 h), une respiration déprimée (0,5 à 3 h) et une hypothermie modérée (0,5 h);
- CRL 41 092:
- la DL-O est supérieure à 256 mg/kg par voie I.P. chez la souris mâle,

- par voie I.P. chez la souris, la dose de 32 mg/kg provoque une hypothermie modérée et la dose de 128 mg/kg provoque une sédation (pendant 0,5 h) et une diminution de la réaction de peur, de la réactivité au toucher et du tonus musculaire; et chez le rat par voie I.P. la dose de 64 mg/kg provoque une sédation fugace avec diminution de la réactivité au toucher et du tonus musculaire.

Les résultats relatifs aux essais sur le système cardiovasculaire ont été résumés ci-après.

- CRL 40 903

Etudié par voie intraduodénale, puis intraveineuse, chez le chien anesthésié, le CRL 40 903 n'a pas d'action sur la pression artérielle; il est tachycardisant à partir de 1 mg/kg; il augmente le débit fémoral à partir de 2,5 mg/kg (maximum + 150 % à 20 mg/kg); la vasodilatation qu'il induit n'est pas complétement supprimée par le propanolol; il ne modifie pas l'hypertension à la noradrénaline et il diminue l'hypotension à l'isoprénaline.

- CRL 40 910

Le CRL 40 910, par voie intraduodénale chez le chien anesthésié, augmente les débits fémoral et vertébral de façon importante, à des doses très faibles (0,5 mg/kg); la tachycardie apparaît à 1 mg/kg, mais elle reste faible; le CRL 40 910 stimule la respiration chez le chien; les températures rectale et cutanée s'élèvent progressivement, ainsi que la pression veineuse; le CRL 40 910 ne modifie pas les effets de l'isoprénaline, il diminue l'hypertension à la noradrénaline; les effets du CRL 40 910 sur la fréquence cardiaque et le débit vertébral sont antagonisés par le propanolol à 1 mg/kg I.V., par contre, l'augmentation du débit fémoral par le CRL 40 910 n'est pas totalement inhibée par le propanolol.

- CRL 41 092

Chez le chien anesthésié, administré par voie intraduodénale, le CRL 41 092 augmente fortement le débit fémoral à partir de 1 mg/kg; on observe une diminution du débit vertébral ainsi que des températures rectale et cutanée; le CRL 41 092 ne modifie pas la fréquence cardiaque et il a tendance à augmenter la pression artérielle; le CRL 41 092 est très légèrement $\beta_1$ et $\beta_2$; il augmente l'hypertension à la noradrénaline; le propanolol, injecté en ffin d'essai à un chien, diminue la fréquence cardiaque et le débit fémoral très légérement dûs au CRL 41 092, le débit fémoral restant toutefois supérieur à sa valeur témoin.

En clinique on a obtenu de bons résultats chez l'homme avec les CRL 40 875 (exemple 1), CRL 40 912 (exemple 2) et CRL 40 897 (exemple 3) dans le traitement des dépressions.

**TABLEAU I**

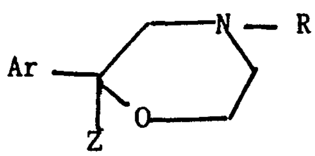

,HCl

| PRODUIT | N° DE CODE | Ar | R | Z | $F_{inst.}$ |
|---|---|---|---|---|---|
| Ex. 1 | CRL 40 875 | p-tolyl | $CH(CH_3)_2$ | H | 225° C (a) |
| Ex. 2 | CRL 40 912 | m-tolyl | $CH_3$ | H | 159° C |
| Ex. 3 | CRL 40 897 | p-tolyl | $CH_3$ | H | 186° C |
| Ex. 4 | CRL 40 903 | p-tolyl | $C(CH_3)_3$ | H | 228° C |
| Ex. 5 | CRL 40 910 | o-tolyl | $C(CH_3)_3$ | H | 230° C |
| Ex. 6 | CRL 41 092 | m-$(CH_3)$-$C_6H_4$ | $CH_2CH_3$ | OH | 194° C |
| CP-1 (b) | CRL 40 915 | o-tolyl | $CH_3$ | H | 192° C (a) |

Notes: (a): avec décomposition

(b): produit de comparaison decrit dans EP-A- 80 940

**Revendications**

pour les Etats contaractants: BE, CH, DE, FR, GB, IT, LI, LU, NL, SE

1. Dérivés de 2-tolyl-morpholine répondant à la formule générale

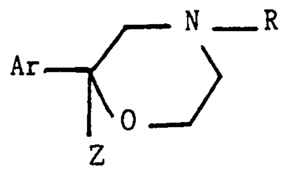

(I)

(où Ar est un groupe tolyle ou trifluorométhylphényle, Z est H ou OH, et R est un groupe alkyle en $C_1$-$C_4$), caractérisé en ce qu'il est choisi parmi l'ensemble des 2-tolyl-4-alkyl-morpholines constitué par

a) la 2-p-tolyl-4-isopropyl-morpholine et ses sels d'addition,
b) la 2-m-tolyl-4-méthyl-morpholine et ses sels d'addition,
c) la 2-p-tolyl-4-méthyl-morpholine et ses sels d'addition.
d) la 2-o-tolyl-4-tertiobutyl-morpholine et ses sels d'addition,
e) la 2-p-tolyl-4-tertiobutyl-morpholine et ses sels d'addition, et,
f) la 2-(m-trifluorbméthylphényl)-2-hydroxy-4-éthyl-morpholine et ses sels d'addition.

2. 2-p-Tolyl-4-isopropyl-morpholine et ses sels d'addition,
3. 2-m-Tolyl-4-méthyl-morpholine et ses sels d'addition,
4. 2-p-Tolyl-4-méthyl-morpholine et ses sels d'addition.
5. 2-p-Tolyl-4-tertiobutyl-morpholine et ses sels d'addition.
6. 2-o-Tolyl-4-tertiobutyl-morpholine et ses sels d'addition.
7. 2-(m-Triflouorométhylphényl)-2-hydroxy-4-éthyl-morpholine et ses sels d'addition.
8. Composition thérapeutique caractérisée en ce qu'elle renferme, en association avec un excipient physiologiquement acceptable, au moins un dérivé de 2-tolyl-4-alkyl-morpholine choisi parmi l'ensemble constitué par les 2-p-tolyl-4-isopropyl-morpholine, 2-m-tolyl-4-méthyl-morpholine, 2-p-tolyl-4-méthyl-morpholine, 2-o-tolyl-4-tertiobutyl-morpholine,2-p-tolyl-4-tertiobutyl-morpholine, 2-(m-trifluorométhylphényl)-2-hydroxy-4-éthyl-morpholine et leurs sels d'addition.
9. Procédé de préparation d'un dérivé de formule I selon la revendication 1 et choisi parmi l'ensemble constitué par

a) la 2-p-tolyl-4-isopropyl-morpholine,
b) la 2-m-tolyl-4-méthyl-morpholine,
c) la 2-p-tolyl-4-méthyl-morpholine,
d) la 2-o-tolyl-4-tertiobutyl-morpholine, et,
e) la 2-p-tolyl-4-tertiobutyl-morpholine et de leurs sels d'addition;

ledit procédé étant caractérisé en ce que l'on cyclise un 2-(N-alkyl-N-β-hydroxyéthyl)-amino-1-tolyl-1-éthanol de formule

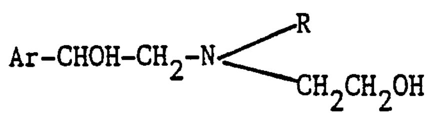

(II)

(où Ar est un groupe tolyle et R est défini comme indiqué dans la revendication 1 ci-dessus) au moyen de $H_2SO_4$ concentré, à une température comprise entre 0° C et 25° C, pendant au moins 1 h.

10. Procédé de préparation d'un dérivé de formule I selon la revendication 1, où Ar est m-trifluorométhylphényle, Z est OH, et R est $CH_2CH_3$, et de ses sels d'addition, ledit procédé étant caractérîsé en ce que l'on fait réagir le bromure de m-trifluorométhylhénacyle de formule

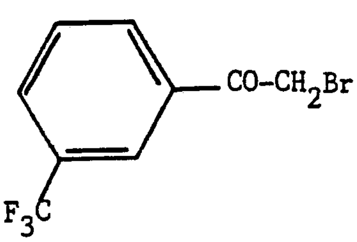

(III Bis)

avec une N-β-hydroxyéthyl-alkylamine de formule $HNRCH_2CH_2OH$ (IV), où R est $CH_2CH_3$, dans un solvant inerte notamment choisi parmi l'éther diméthylique et l'éther diéthylique, à raison de 2 moles environ de (III Bis) pour 1 mole de (IV), la durée de la réaction étant comprise entre 4 et 24 h, et la température entre 5 et 25° C.

**Revendications**

pour l'Etat contractant: AT

Procédé de préparation d'un dérivé de 2-tolyl-morpholine répondant à la formule générale

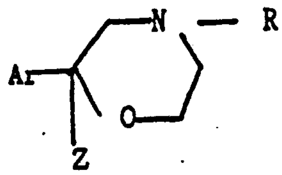

(I)

(où Ar est un groupe tolyle ou trifluorométhylphényle, Z est R ou OH, et R est un groupe alkyle en $C_1$-$C_4$ et choisi parmi l'ensemble des 2-tolyl-4-tolyl-morpholines constitué par
a) la 2-p-tolyl-4-isopropyl-morpholine,
b) la 2-m-tolyl-4-méthyl-morpholine,
c) la 2-p-tolyl-4-méthyl-morpholine,
d) la 2-o-tolyl-4-tertiobutyl-morpholine,
e) la 2-p-tolyl-4-tertiobutyl-morpholine,
f) la 2-(m-triflouorométhylphényl)-2-hydroxy-4-éthyl-morpholine et leurs sels l'addition.
ledit procédé étant caractérisé en ce que
1.) pour préparer un composé dans lequel Z est H, l'on cyclise un 2-(N-alkyl-N-β-hydroxyéthyl)-amino-1-tolyl-1-éthanol de formule

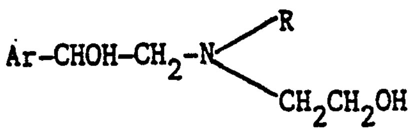

(II)

(où Ar est un groupe tolyle et R est défini comme indiqué ci-dessus) au moyen se $H_2SO_4$ concentré à une température comprise entre 0° C et 25° C, pendant au moins 1 h,
et en ce que
2.) pour préparer un composé dans lequel Z est OH, Ar est m-trifluorométhylphényle et R est $CH_2CH_3$, l'on fait réagir le bromure de m-trifluorométhyl-phénacyle de formule

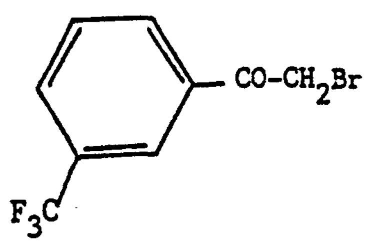

(III Bis)

avec une N-β -hydroxyéthyl-alkylamine de formule $NHRCH_2CH_2OH$ (IV), où R est $CH_2CH_3$, dans un solvant inerte notamment choisi parmi l'éther diméthylique et l'éther diéthylique, à raison de 2 moles environ de (III Bis) pour 1 mole de (IV) la durée de la rèaction étant comprise entre 4 et 24 h, et la température entre 5 et 25° C.

2. Procédé de préparation d'un dérivé de 2-tolyl-morpholine répondant à la formule générale

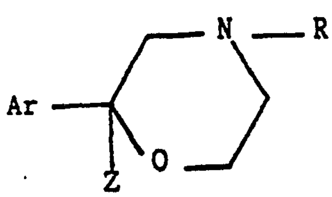

(I)

où Ar est un groupe tolyle Z est H et R est $CH_3$, $CH(CH_3)_2$ ou $C(CH_3)_3$, et choisi parmi l'ensemble des 2-tolyl-4-alkyl-morpholines constitué par
a) la 2-p-tolyl-4-isopropyl-morpholine,
b) la 2-m-tolyl-4-méthyl-morpholine,
c) la 2-p-tolyl-4-méthyl-morpholine,
d) la 2-p-tolyl-4-tertiobutyl-morpholine,

e) la 2-o-tolyl-4-tertiobutyl-morpholine
et leurs sels d'addition;
ledit procédé étant caractérisé en ce que l'on cyclise un 2-(N-alkyl-N-β-hydroxyléthyl)-amino-1-tolyl-1-éthanol de formule

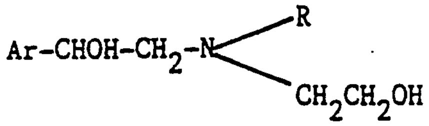

(II)

[ù Ar est un groupe tolyle et R est $CH_3$, $CH(CH_3)_2$ ou $C(CH_3)_3$ comme indiqué ci-dessus] au moyen de $H_2SO_4$ concentré, à une température comprise entre 0° C, pendant au moins 1 h,

3. Procédé de préparation de la 2-(m-triflourométhylphényl)-2-hydroxy-4-éthyl-morpholine et de ses sels d'addition, caractérisé en ce que l'on fait réagri le bromure de m-triflourométhyl-phénacyle de formule

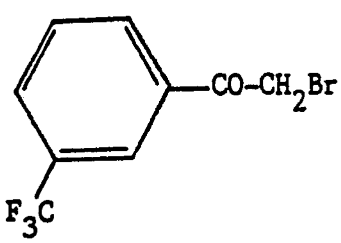

(III Bis)

avec une N-β-hydroxyéthyl-alkylamine de formule $NHRCH_2CH_2OH$ (IV), où R est $CH_2CH_3$, dans un solvant inerte notamment choisi parmi l'éther diméthylique et l'éther diéthylique, à raison de 2 moles environ de (III Bis) pour 1 mole de (IV), la durée de la réaction étant comprise entre 4 et 24 h, et la température entre 5 et 25° C.

**Patentansprüche**

für die Vertragsstaaten BE, CH, DE, FR, GB, IT, LI, LU, NL, SE.

1. 2-Tolyl-morpholin-derivate der allgemeinen Formel:

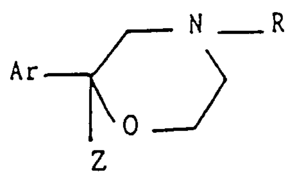

(I),

in welcher Ar eine Tolyl- oder Triflourmethylphenylgruppe bedeutet, Z für H oder OH steht und R eine $C_1$-$C_4$-Alkylgruppe darstellt, dadurch gekennzeichnet, daß sie aus der von 2-Tolyl-4-alkyl-morpholinen, bestehend aus a) 2-p-Tolyl-4-isopropyl-morpholin und seinen Additionssalzen, b) 2-m-Tolyl-4-methyl-morpholin und seinen Additionssalzen c) 2-p-Tolyl-4-methyl-morpholin und seinen Additionssalzen, d) 2-o-Tolyl-4-tert.bu-tyl-morpholin und seinen Additionssalzen, e) 2-p-To-lyl-4-tert.butyl-morpholin und seinen Additionssalzen und f) 2-(m-Trifluormethylphenyl)-2-hydroxy-4-ethyl-morpholin und seinen Additionssalzen, ausgewählt sind.

2. 2-p-Tolyl-4-isopropyl-morpholin und seine Additionssalze.

3. 2-m-Tolyl-4-methyl-morpholin und seine Additionssalze.

4. 2-p-Tolyl-4-methyl-morpholin und seine Additionssalze.

5. 2-p-Tolyl-4-tert.butyl-morpholin und seine Additionssalze.

6. 2-o-Tolyl-4-tert.butyl-morpholin und seine Additionssalze.

7. 2-(m-Trifluormethylphenyl)-2-hydroxy-4-ethyl-mor-pholin und seine Additionssalze.

8. Therapeutische Mittel, dadurch gekennzeichnet, daß sie in Verbindung mit einem physiologisch zulässigen Bindemittel mindestens ein 2-Tolyl-4-alkyl-mor-pholin-derivat, ausgewählt aus der Gruppe, bestehend aus 2-p-Tolyl-4-isopropyl-morpholin, 2-m-Tolyl-4-me-thyl-morpholin 2-p-Tolyl-4-methyl-morpholin, 2-o-To-lyl-4-tert.butyl-morpholin, 2-p-Tolyl-4-tert.butyl-morpholin, 2-(m-Trifluormethylphenyl)-2-hydroxy-4-ethyl-morpholin und deren Additionssalzen, enthalten.

9. Verfahren zur Herstellung eines Derivates der Formel I nach Anspruch 1, und ausgewählt aus der Gruppe, bestehend aus

a) 2-p-Tolyl-4-isopropyl-morpholin, b) 2-m-Tolyl-4-methyl-morpholin, c) 2-p-Tolyl-4-methyl-morpholin, d) 2-o-Tolyl-4-tert.butyl-morpholin und e) 2-p-To-lyl-4-tert.butyl-morpholin und deren Additionssalzen, dadurch

gekennzeichnet, daß man ein 2-(N-Alkyl-N-β-hydroxyethyl)-amino-1-tolyl-1-ethanol der Formel:

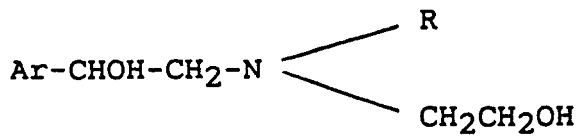

(II),

in welcher Ar eine Tolylgruppe darstellt und R die obige Bedeutung hat, mittels konz. $H_2S0_4$ bei einer Temperatur zwischen O° und 25° C über einen Zeitraum von mindestens 1 Stunde cyclisiert.

10. Verfahren zur Herstellung eines Derivates der Formel I nach Anspruch 1, in welcher Ar m-Trifluormethylphenyl bedeutet, Z für OH steht und R für $CH_2H_3$ steht, und von seinen Additionssalzen, dadurch gekennzeichnet, daß man m-Trifluormethyl-phenacyl-bromid der Formel:

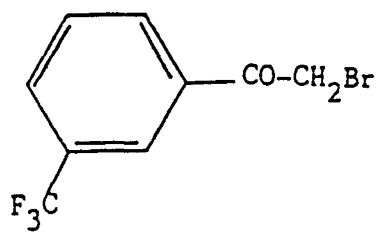

(III Bis)

mit einem N-β-Hydroxyethyl-alkylamin der Formel $HNRCH_2CH_2OH$ (IV), in welcher R für $CH_2CH_3$ steht, in einem inerten Lösungsmittel, insbesondere ausgewählt aus Dimethylether und Diethylether, in einer Menge von etwa 2 Mol einer Verbindung der Formel (III Bis) pro 1 Mol einer Verbindung der Formel (IV) über einen Zeitraum von 4 bis 24 Stunden bei einer Temperatur zwischen 5 und 25° C umsetzt.

**Patentansprüche**

für den Vertragsstatt AT:

1. Verfahren zur Herstellung von 2-Tolyl-morpholin-derivaten der allgemeinen Formel:

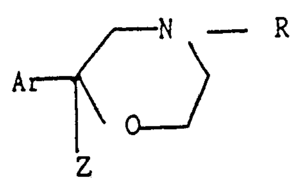

(I),

in welcher Ar eine Tolyl- oder Trifluormethylphenylgruppe bedeutet, Z für H oder OH steht und R eine $C_1$-$C_4$-Alkylgruppe darstellt, und ausgewählt aus der Gruppe von 2-Tolyl-4-alkyl-morpholinen, bestehend aus

a) 2-p-Tolyl-4-isopropyl-morpholin, b) 2-m-Tolyl-4-methyl-morpholin, c) 2-p-Tolyl-4-methyl-morpholin, d) 2-o-Tolyl-4-tert.butyl-morpholin, e) 2-p-Tolyl-4-tert.butyl-morpholin, f) 2-(m-Trifluormethylphenyl)-2-hydroxy-4-ethyl-morpholin und deren Additionssalzen, dadurch gekennzeichnet, daß man

1) zur Herstellung von Verbindungen der allgemeinen Formel (I), in welcher Z für H steht, ein 2-(N-Al-kyl-N-β-hydroxy-ethyl)-amino-1-tolyl-1-ethanol der allgemeinen Formel:

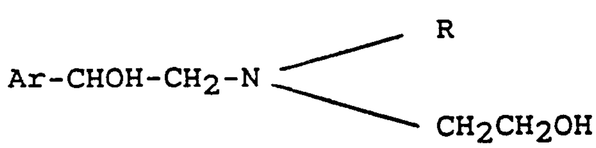

(II),

in welcher Ar eine Tolylgruppe darstellt und R die obige Bedeutung hat, mittels konz. $H_2SO_4$ bei einer Temperatur zwischen 0° C und 25° C über einen Zeitraum von mindestens 1 Stunde cyclisiert, und daß man

2) zur Herstellung von Verbindungen der allgemeinen Formel (I), in welcher Z für OH, Ar für m-Trifluormethylphenyl und R für $CH_2CH_3$ stehen, m-Trifluormethyl-phenacyl-bromid der Formel:

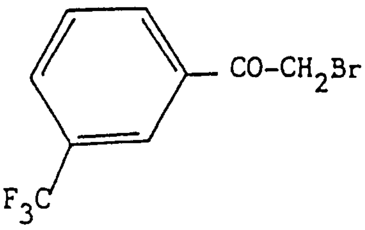

(III Bis)

mit einem N-β-Hydroxyethyl-alkylamin der allgemeinen Formel:

$NHRCH_2CH_2OH$ (IV),

in welcher R für $CH_2CH_3$ steht, in einem inerten Lösungsmittel, insbesondere ausgewählt aus Dimethylether und Diethylether, in einer Menge von etwa 2 Mol einer Verbindung der allgemeinen Formel (III Bis) pro 1 Mol der Verbindung der allgemeinen Formel (IV) über einen Zeitraum von 4 bis 24 Stunden bei einer Temperatur zwischen 5 und 25° C umsetzt.

2. Verfahren nach Anspruch 1 zur Herstellung von 2-Tolyl-morpholin-derivaten der allgemeinen Formel:

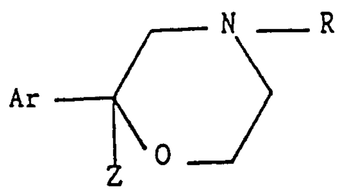

(I)

in welcher Ar eine Tolylgruppe bedeutet Z für H steht und R für $CH_3$ $CH(CH_3)_2$ oder $C(CH_3)_3$ steht, und ausgewählt aus der Gruppe von 2-Tolyl-4-alkyl-morpholinen, bestehend aus

a) 2-p-Tolyl-4-isopropyl-morpholin, b) 2-m-Tolyl-4-methyl-morpholin c) 2-p-Tolyl-4-methyl-morpholin, d) 2-p-Tolyl-4-tert.butyl-morpholin, e) 2-o-Tolyl-4-tert.butyl-morpholin und deren Additionssalzen, dadurch gekennzeichnet, daß man ein 2-(N-Alkyl-N-β-hy-droxyethyl)-amino-1-tolyl-1-ethanol der allgemeinen Formel:

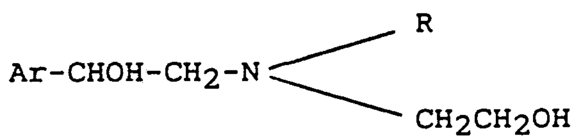

(II),

in welcher Ar eine Tolylgruppe bedeutet und R für $CH_3$, $CH(CH_3)_2$ oder $C(CH_3)_3$ steht, mittels konz. $H_2SO_4$ bei einer Temperatur zwischen 0° C und 25° C über einen Zeitraum von mindestens 1 Stunde cyclisiert.

3. Verfahren nach Anspruch 1 zur Herstellung von 2-(m-Trifluormethylphenyl)-2-hydroxy-4-ethyl-morpholin und von seinen Additionssalzen, dadurch gekennzeichnet, daß man m-Trifluormethyl-phenacyl-bromid der Formel:

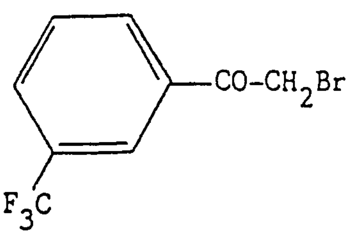

(III Bis)

mit einem N-β-Hydroxyethyl-alkylamin der allgemeinen Formel

$NHRCH_2CH_2OH$ (IV),

in welcher R für $CH_2CH_3$ steht, in einem inerten Lösungsmittel, insbesondere ausgewählt aus Dimethylether und Diethylether, in einer Menge von etwa 2 Mol der Verbindung der Formel (III Bis) pro 1 Mol der Verbindung der allgemeinen Formel (IV) über einen Zeitraum von 4 bis 24 Stunden bei einer Temperatur zwischen 5 und 25° C umsetzt.

**Claims**

for the contracting states: BE, CH, DE, FR, GB, IT, LI, LU, NL, SE

1. A 2-tolylmorpholine derivative corresponding to the general formula

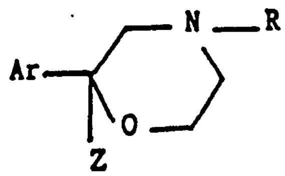

(I)

(in which Ar is a tolyl or trifluoromethylphenyl group, Z is H or OH and R is a $C_1$-$C_4$ alkyl group), which is selected from the group of 2-tolyl-4-alkylmorpholines consisting of
a) 2-p-tolyl-4-isopropylmorpholine and its addition salts,
b) 2-m-tolyl-4-methylmorpholine and its addition salts,
c) 2-p-tolyl-4-methylmorpholine and its addition salts,
d) 2-o-tolyl-4-tert.-butylmorpholine and its addition salts,
e) 2-p-tolyl-4-tert.-butylmorpholine and its addition salts, and
f) 2-(m-trifluoromethylphenyl)-2-hydroxy-4-ethylmorpholine and its addition salts.

2. 2-p-Tolyl-4-isopropylmorpholine and its addition salts.

3. 2-m-Tolyl-4-methylmorpholine and its addition salts.

4. 2-p-Tolyl-4-methylmorpholine and its addition salts.

5. 2-p-Tolyl-4-tert.-butylmorpholine and its addition salts.

6. 2-o-Tolyl-4-tert.-butylmorpholine and its addition salts.

7. 2-(m-Trifluoromethylphenyl)-2-hydroxy-4-ethylmorpholine and its addition salts.

8. A therapeutic composition which contains, in association with a physiologically acceptable excipient, at least one 2-tolyl-4-alkylmorpholine derivative selected from the group consisting of 2-p-tolyl-4-isopropylmorpholine, 2-m-tolyl-4-methylmorpholine, 2-p-tolyl-4-methylmorpholine, 2-o-tolyl-4-tert.-butylmorpholine, 2-p-tolyl-4-tert.-butylmorpholine, 2-(m-trifluoromethylphenyl)-2-hydroxy-4-ethylmorpholine and their addition salts.

9. A method for the preparation of a derivative of the formula I as claimed in claim 1 and selected from the group consisting of
a) 2-p-tolyl-4-isopropylmorpholine,
b) 2-m-tolyl-4-methylmorpholine,
c) 2-p-tolyl-4-methylmorpholine,
d) 2-o-tolyl-4-tert.-butylmorpholine and
e) 2-p-tolyl-4-tert.-butylmorpholine
and their addition salts,
in which method a 2-(N-alkyl-N-β-hydroxyethyl)amino-1-tolylethanol of the formula

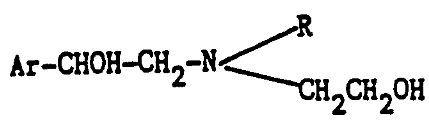

(II)

(in which Ar is a tolyl group and R is defined as indicated in claim 1 above) is cyclized by means of concentrated $H_2SO_4$, at a temperature of between 0° C and 25° C, for at least 1 hour.

10. A method for the preparation of a derivative of the formula I as claimed in claim 1 in which Ar is m-trifluoromethylphenyl, Z is OH and R is $CH_2CH_3$, and of its addition salts, in which method m-trifluoromethylphenacyl bromide of the formula

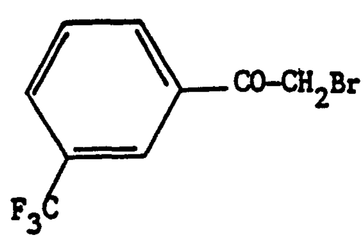

(III bis)

is reacted with an N-β-hydroxyethylalkylamine of the formula $HNRCH_2CH_2OH$ (IV) (in which R is $CH_2CH_3$), in an inert solvent selected especially from dimethyl ether and diethyl ether, at a rate of about 2 mol of (III bis) per

mol of (IV) the reaction time being between 4 and 24 hours and the temperature being between 5 and 25°C.

## Claims

for the contracting state: AT

1. A Method for the preparation of a 2-tolylmorpho-line derivative corresponding to the general formula

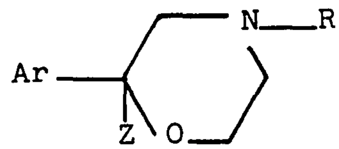

(I)

(in which Ar is a tolyl or trifluoromethylphenyl group Z is H or OH and R is a $C_1$-$C_4$ alkyl group), which is selected from the group of 2-tolyl-4-alkylmorpholines consisting of

a) 2-p-tolyl-4-isopropylmorpholine,
b) 2-m-tolyl-4-methylmorpholine,
c) 2-p-tolyl-4-methylmorpholine,
d) 2-o-tolyl-4-tert.-butylmorpholine,
e) 2-p-tolyl-4-tert.-butylmorpholine, and
f) 2-(m-trifluoromethylphenyl)-2-hydroxy-4-ethylmorpholine and their addition salts,

said method being characterized in that

1. for preparing a derivative in which Z is H, a 2-(N-alkyl-N-β-hydroxyethyl)-amino-1-tolylethanol of the formula

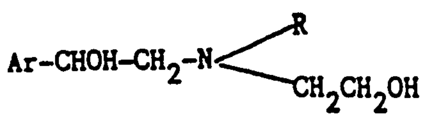

(II)

(in which Ar is a tolyl group and R is defined as indicated above) is cyclized by means of concentrated $H_2SO_4$ at a temperature of between 0°C and 25°C, for at least 1 hour, and

2. for preparing a derivative in which Z is OH, Ar is m-trifluoromethylphenyl and R is $CH_2CH_3$, m-trifluoromethylphenacyl bromide of the formula

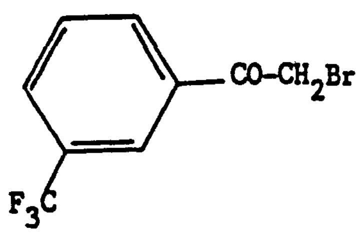

(III bis)

is reacted with an N-β-hydroxyethylalkylamine of the formula $HNRCH_2CH_2OH$ (IV) (in which R is $CH_2CH_3$), in an inert solvent selected especially from dimethyl ether and diethyl ether, at a rate of about 2 mol of (III bis) per mol of (IV) the reaction time being between 4 and 24 hours and the temperature being between 5 and 25°C.

2. A method for preparing a 2-tolylmorpholine derivative of the general formula

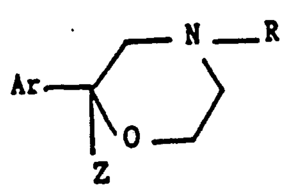

(I)

[in which Ar is a tolyl group, Z is H and R is $CH_3$,$CH(CH_3)_2$ or $C(CH_3)_3$], which is selected from the group of 2-tolyl-4-alkylmorpholines consisting of

a) 2-p-tolyl-4-isopropylmorpholine,

b) 2-m-tolyl-4-methylmorpholine;
c) 2-p-tolyl-4-methylmorpholine,
d) 2-o-tolyl-4-tert.-butylmorpholine and
e) 2-p-tolyl-4-tert.-butylmorpholine
and their addition salts,
characterized in that a 2-(N-alkyl-N-β-hydroxyethyl)amino-1-tolylethanol of the formula

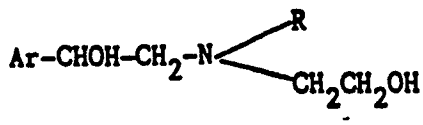

(II)

[in which Ar is a tolyl group and R is $CH_3$,$CH(CH_3)_2$ or $C(CH_3)_3$ above] is cyclized by means of concentrated $H_2SO_4$, at a temperature of between 0° and 25° C for at least 1 hour.

3. A method for preparing 2-(m-trifluoromethyl-phenyl-2-hydroxy-4-ethylmorpholine and its addition salts characterized in that m-trifluoromethylphenacyl bromide of the formula

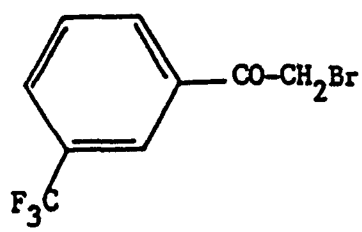

(III bis)

is reacted with an N-β-hydroxyethylalkylamine of the formula $HNRCH_2CH_2OH$ (IV) (in which R is $CH_2CH_3$), in an inert solvent selected especially from dimethyl ether and diethyl ether, at a rate of about 2 mol of (III bis) per mol of (IV), the reaction time being between 4 and 24 hours and the temperature being between 5 and 25° C.